# EUROPÄISCHE PATENTSCHRIFT

(11) **EP 3 400 997 B1**
(45) Veröffentlichungstag und Bekanntmachung des Hinweises auf die Patenterteilung: **08.07.2020**
(21) Anmeldenummer: 18170425.5
(22) Anmeldetag: 02.05.2018
(51) Int. Cl.: B01D 25/164, B01D 25/21, B01D 25/28

(54) **PLATTE FÜR EINE FILTERPRESSE, FILTERPRESSE, VERWENDUNG DER FILTERPRESSE UND VERFAHREN ZUR REINIGUNG DER FILTERPRESSE**
PLATE FOR A FILTER PRESS, FILTER PRESS, USE OF THE FILTER PRESS AND METHOD FOR CLEANING THE FILTER PRESS
PLAQUE POUR UN FILTRE-PRESSE, FILTRE-PRESSE, UTILISATION DU FILTRE-PRESSE ET PROCÉDÉ DE NETTOYAGE DU FILTRE-PRESSE

(30) Priorität: 04.05.2017 DE 102017207484
(43) Veröffentlichungstag der Anmeldung: 14.11.2018
(73) Patentinhaber: Strassburger Filter GmbH & Co. KG, 67593 Westhofen /Rheinhessen (DE)
(72) Erfinder: Sebastian, Jürgen, 67593 Westhofen (DE)
(74) Vertreter: Mehler Achler

(56) Entgegenhaltungen:
- DE-A1- 3 222 988
- DE-A1- 10 221 061
- US-A- 4 891 117

## Beschreibung

Die Erfindung betrifft eine Platte für eine Filterpresse, eine Filterpresse, die Verwendung einer solchen Filterpresse sowie ein Verfahren zur Reinigung der Platten einer solchen Filterpresse.

Filterpressen werden in sogenannte Rahmenfilterpressen, Kammerfilterpressen und Membranfilterpressen unterschieden. Hierbei handelt es sich um die gleiche Ausführung des Filterpressgestells das jedoch mit unterschiedlichen Plattenarten bestückt ist (siehe z.B. Horst Gasper "Handbuch der industriellen Fest/Flüssig-Filtration", Hüthig-Verlag Heidelberg 1990, S. 104 - 111).

Ihren Einsatz finden Filterpressen vor allem in der Getränke- und Lebensmittelindustrie sowie in der Kosmetik, Chemie und Pharmaindustrie. Dabei werden insbesondere in der Pharmaindustrie höchste Reinheitsanforderungen und ein GMP-gerechtes (*Good Manufacturing Practice*) Design an die Filtrationssysteme gestellt. Bei der GMP handelt es sich um Qualitätsansprüche, die während der Herstellung, Verarbeitung, Verpackung und Lagerung von Arzneimitteln eingehalten werden müssen.

Die DE 102 21 061 A1 offenbart eine Plattenanordnung, bei der abwechselnd Filterplatten und Membranplatten angeordnet sind. Die Filterplatte ist an beiden Seitenflächen mit einem Filtertuch bestückt, welches jeweils in einer Aussparung des Grundkörpers der Filterplatte befestigt ist. Die Membranplatte weist auf beiden Seiten jeweils eine Membran auf, die in jeweils einer Aussparung auf beiden Seiten des Grundkörpers der Membranplatte befestigt ist.

Bei der Filterpresse ist zwischen dem Filtertuch der Filterplatte und der benachbarten Membran einer Membranplatte eine Filterkammer ausgebildet. Zwischen der Membran und dem Grundkörper der Membranplatte ist eine Druckkammer ausgebildet. Über die Membranplatte kann beidseitig beim Zusammenbau der Filterpresse jeweils ein weiteres Filtertuch gelegt werden.

Die Filtertücher erstrecken sich jeweils bis zum Rand der Filter- und Membranplatten und werden dort beim Schließen der Filterpresse gegeneinander gedrückt und auf diese Weise gehalten. Dadurch, dass die Filtertücher am Rand der Platten enden, besteht die Gefahr, dass Flüssigkeit aus diesem Bereich heraustropft, wenn nicht zusätzliche Dichtelemente vorgesehen werden.

Zum Reinigen der Filterpresse wird die Filterpresse geöffnet und die Filterkuchen werden nach unten ausgetragen. Die einzelnen Filter- und Membranplatten werden entnommen und in eine außerhalb der Filterpresse angeordneten Reinigungsvorrichtung gereinigt. Beim Öffnen der Filterpresse müssen die Filtertücher separat entnommen und gereinigt werden.

Bei der Verwendung von Filterschichten anstelle von Filtertüchern müssen die Filterschichten entsorgt und durch neue Filterschichten ersetzt werden.

Aus der WO 2006/015637 A1 ist eine Filtervorrichtung mit einer vorgebbaren Anzahl an Filter- und Trubrahmen mit einer Dichtungseinrichtung bekannt. Die Filter- und Trubrahmen sind in alternierender Reihenfolge angeordnet, wobei die Filterrahmen zumindest auf ihrer dem jeweiligen Trubrahmen zugewandten Seite ein Filtermittel aufweisen, welches randseitig in Klemmspalten zwischen zwei jeweiligen benachbarten Rahmen festlegbar ist.

Zwischen der Dichteinrichtung und dem Klemmspalt ist ein Verbindungsraum angeordnet, der in einen zentralen Verbindungskanal mündet, welcher die Zu- und Abfuhr von Reinigungsmedien ermöglicht.

Der Reinigungsvorgang bei dieser Filtervorrichtung sieht vor, dass die Klemmspalte zunächst geöffnet und das Filtermedium durch die Klemmspalte entfernt wird. Anschließend werden die Platten wieder dichtend zusammengepresst und die Filtervorrichtung wird ähnlich dem Filterbetrieb entsprechend im Abreinigungsbetrieb unter der Verwendung von Reinigungsmedien und/oder Spülmedien und/oder Sterilisationsmedien gefahren.

Nach dem Reinigungsvorgang muss die Filterpresse erneut geöffnet werden, damit frische Filtermedien eingesetzt werden können. Dabei kann es zu einer Kontaminierung des Innenraums mit Schmutzpartikeln kommen.

Immer häufiger werden Filtermedien in Form von Filtertüchern verwendet, die nach einer Reinigung bis zu 100-mal wiederverwendet werden können.

Die DE 10 2012 209 591 A1 beschreibt daher eine Filterpresse mit einer Plattenanordnung bestehend aus Membranplatten, Filterplatten und Rahmenplatten, wobei Filtertücher mittels Halteelementen in Form von Haltezapfen in den Randbereichen der Seitenflächen von Membran- und Filterplatten lösbar befestigt sind.

Auf diese Weise können die wiederverwendbaren Filtertücher während der Reinigung in der Reinigungsvorrichtung an den Membranplatten bzw. den Filterplatten verbleiben und müssen nicht wie in den zuvor beschriebenen Schriften vor der Reinigung entfernt werden.

Aufgabe der vorliegenden Erfindung ist es, eine Platte für eine Filterpresse, eine Filterpresse und ein Verfahren zur Reinigung und/oder Sterilisation zur Verfügung zu stellen, welche es erlauben, eine Reinigung der Filterpresse und ihrer Komponenten durchzuführen, die den hohen Qualitätsstandards insbesondere im Bereich der Pharmaindustrie genügt, und eine Reinigung und Sterilisation all der Bereiche auf einfache Weise erlaubt, die mit dem filtrierten Produkt in Berührung kommen.

Gelöst wird diese Aufgabe mit einer Platte für eine Filterpresse, einer Filterpresse und der Verwendung einer Filterpresse gemäß den Ansprüchen 1, 12 und 13 sowie einem Verfahren zur Reinigung und/oder Sterilisation einer Filterpresse gemäß Anspruch 14 und 15.

Die Platte für eine Filterpresse ist gekennzeichnet durch
- einen Grundkörper, der aus einem ersten Material mit einer Härte H₁ besteht und eine äußere Umfangsfläche aufweist, und
- einen an der äußeren Umfangsfläche angeordneten Rahmen, der aus einem zweiten Material mit einer Härte H₂ besteht, wobei H₂ < H₁ ist, und
- wobei der Rahmen an mindestens einer Seitenfläche mindestens eine gegenüber der Seitenfläche vorstehende, umlaufende Dichtung aufweist.

Die Verwendung von zwei unterschiedlich harten Materialien für unterschiedliche Bereiche der Platte hat den Vorteil, dass die Materialien bezüglich ihrer Eigenschaften für den Filtrationsbereich einerseits und den Dichtbereich andererseits optimal eingestellt werden können. Der Grundkörper ist während der Filtration hohen Drücken ausgesetzt, so dass der Grundkörper aus hartem und damit stabilen Material gefertigt werden kann. Der Rahmen wird aus weicherem Material hergestellt, das beim Zusammenpressen der Platten aufgrund seiner Flexibilität die notwendige Dichtfunktion übernimmt.

Es ist somit möglich, die umlaufende Dichtung, die an mindestens einer Seitenfläche des Rahmens gegenüber der Seitenfläche vorsteht, aus demselben Material wie den Rahmen zu fertigen.

In einer vorteilhaften Ausführung ist die Dichtung und der Rahmen aus einem Stück gefertigt.

Aufgrund der hohen Reinheits- und Qualitätsanforderungen sind Fugen und scharfe Kanten oder Lücken zu vermeiden. Die Fertigung des Rahmens und der Dichtung aus einem Stück sowie der Verbund von Grundkörper und flexiblem Rahmen verhindert daher das Auftreten von fertigungsbedingten Fugen, Kanten und/oder Leckagen, in oder an denen sich während der Verwendung der Platte in einer Filterpresse Verunreinigungen ansammeln können, die bei einem Reinigungsvorgang nur sehr schlecht bzw. gar nicht zu erreichen sind.

Vorzugsweise ist der Rahmen mit seiner umlaufenden inneren Umfangsfläche mit der umlaufenden äußeren Umfangsfläche des Grundkörpers verbunden.

In einer bevorzugten Ausführungsform ist die innere Umfangsfläche des Rahmens mit der äußeren Umfangsfläche des Grundkörpers verschweißt.

Gegenüber anderen Fügeverfahren für Kunststoffe, wie beispielsweise dem mechanischen Fügen oder dem Verkleben, bietet das Verschweißen den Vorteil einer hohen Verbindungsqualität und der Dichtheit gegenüber flüssigen Medien.

Insbesondere wenn der Grundkörper der Platte aus Polypropylen besteht, ist eine Schweißverbindung mit einem auf Polypropylen basierenden thermoplastischen Elastomer vorteilhaft, da sich leichter ein gemeinsames Gefüge zwischen beiden Bauteilen ausbildet.

Vorzugsweise weist der Rahmen eine Härte H₂ von 90 bis 100 shore A, besonders bevorzugt 93 bis 95 shore A auf.

Vorteilhaft ist es, wenn der Rahmen ein thermoplastisches Elastomer aufweist oder vollständig aus einem thermoplastischen Polymer, insbesondere aus dem thermoplastischen Elastomer besteht.

Thermoplastische Elastomere sind Kunststoffe, die meist aus einem "weichen" Elastomer wie beispielsweise Ethylen-Propylen-Dien; M-Gruppe (EPDM) und einer harten thermoplastischen Komponente wie zum Beispiel Polypropylen bestehen.

Ein Vorteil dieser Kunststoffe ist zum einen ihre reversible Formveränderung und zum anderen die Möglichkeit diese schweißen zu können, um damit wasserdichte Verbindungen herzustellen.

Mit der obengenannten Härte ist der flexible Rahmen weicher als der Grundkörper, der vorzugsweise aus Polypropylen besteht.

Vorzugsweise weist der Grundkörper eine Härte H₁ von 60 bis 80 Shore D auf. Die Messung der Härten erfolgt gemäß der Norm DIN ISO 7619-1.

Vorzugsweise weist die umlaufende Dichtung im unbelasteten Zustand einen ersten Vorsprung auf, der in einen zweiten Vorsprung übergeht.

Vorzugsweise überragt der zweite Vorsprung den ersten Vorsprung.

Eine derart abgestufte Dichtung hat den Vorteil, dass beim Zusammenpressen einer Plattenanordnung in einer Filterpresse eine definierte Zwischenposition der Platten zueinander eingenommen werden kann. Diese definierte Zwischenposition wird im eingebauten Zustand vorzugsweise für die Sterilisation der Platten verwendet.

Vorzugsweise weist jede zweite Platte der Plattenanordnung den erfindungsgemäßen Aufbau aus Grundkörper und Rahmen mit Dichtung auf.

Im Reinigungszustand und im Filterzustand werden die Platten erfindungsgemäß mit unterschiedlichen Anpressdrücken beaufschlagt. Während im Filterzustand der Anpressdruck mit z. B 240 bar so groß ist, dass die flexible Dichtung in ihrer Gesamtheit zusammengepresst wird und die Platten fest aneinander liegen, ist der Druck für den Reinigungszustand so gewählt, dass die flexible Dichtung nur soweit zusammengepresst ist, bis die benachbarte Platte den ersten Vorsprung erreicht und so ein schmaler Spalt von wenigen Millimetern zwischen benachbarten Platten entsteht, der breit genug ist, um eine sogenannte CIP-Reinigung (Cleaning in Place) durchzuführen. Das CIP-Reinigungsverfahren ist ein bekanntes Verfahren, welches eine Reinigung der Filterpresse an Ort und Stelle beinhaltet, bei der die Filterpresse, wie in der obengenannten Schrift WO 2006/015637 A1 beschrieben, anstatt mit dem zu filternden Medium mit einem Reinigungs- und/oder Sterilisationsmittel betrieben wird.

Vorzugsweise weist der erste Vorsprung eine zur Seitenfläche des Rahmens parallel ausgerichtete Anschlagsfläche auf.

Eine solche parallele Fläche hat den Vorteil, dass beim Zusammenpressen eine definierte Zwischenposition eingestellt werden kann und der Ausgangsdruck gleichmäßig über die gesamte Plattenanordnung verteilt wird. Es wird dadurch eine Druckfortpflanzung über die gesamte Plattenanordnung sichergestellt. Würde man die ersten Vorsprünge der Platten nicht als Anschlag ausgestalten, wären die Dichtungen der ersten Platten, an denen der Anpressdruck angelegt wird, stärker, d. h. über die Zwischenposition hinaus verpresst, während die Dichtungen der Platten am anderen Ende der Filterpresse möglicherweise überhaupt nicht verformt wären.

Üblicherweise weist eine Filterpresse eine Plattenanordnung mit bis zu 120 Filterplatten auf (z. B. 28 Membranplatten, 29 Filterplatten, 56 Rahmenplatten). Die stufenförmige, Dichtung sorgt dafür, dass der für die Reinigung beaufschlagte Anpressdruck gleichmäßig über die gesamte Plattenanordnung verteilt wird und ein definierter Spalt von wenigen Millimetern zwischen allen benachbarten Platten entsteht, der für die Reinigung notwendig ist.

Eine parallel zur Seitenfläche der Platte verlaufende Anschlagfläche des ersten Vorsprungs verleiht diesem einen höheren Verformungswiderstand und ermöglicht eine optimierte Druckfortpflanzung über die gesamte Plattenanordnung hinweg sowie die Bestimmung eines für die Erzeugung der vorgesehenen Spaltbreite notwendigen Drucks. Vorteilhafterweise entspricht die gewünschte Spaltbreite der Höhe des ersten Vorsprungs, welche üblicherweise etwa 1,5 mm beträgt. Aufgrund der parallel zur Seitenfläche ausgerichteten Anschlagfläche des ersten Vorsprungs werden hohe Materialspannungen in der Dichtung vermieden und eine längere Haltbarkeit der Dichtung gewährleistet.

Vorzugsweise ist der erste Vorsprung näher an der äußeren Umfangsfläche des Rahmens angeordnet als der zweite Vorsprung.

Durch die Anordnung des ersten Vorsprungs näher an der äußeren Umfangsfläche des Rahmens als der zweite Vorsprung ist sichergestellt, dass der größere Vorsprung den inneren Kammerbereich begrenzt und keine Fuge entsteht, die für das Reinigungs- und/oder Sterilisationsmedium schlecht erreichbar ist.

Vorteilhafterweise weist die Seitenfläche, die die Dichtung aufweist, eine umlaufende Nut auf, wobei die Nut vorzugsweise an den zweiten Vorsprung angrenzt. Vorzugsweise grenzt die Nut unmittelbar an den zweiten Vorsprung an, was bedeutet, dass die Flanke des zweiten Vorsprungs in die Nutflanke übergeht.

Vorzugsweise hat die Nut eine Tiefe von 0,5 bis 1,5 cm und besonders bevorzugt 0,8 bis 1,2 cm sowie eine Nutbreite von 10-20 mm.
Die Nutbreite wird in der Ebene der Seitenfläche gemessen.

Eine unmittelbar an den zweiten Vorsprung angrenzende Nut erlaubt die Aufnahme des verdrängten Volumens der zusammengepressten Dichtung und vermindert so die auftretenden Materialspannungen im angepressten Zustand und erhöht in der Folge die Lebensdauer der Dichtung. Das Nutvolumen ist daher auf das Volumen der Dichtung abzustimmen.

Die Reihenfolge von erstem und zweitem Vorsprung kann auch vertauscht werden.

In einer vorteilhaften Ausführungsform ist die Platte eine Filterplatte.

In einer weiteren vorteilhaften Ausführungsform ist die Platte eine Membranplatte.

In einer besonders bevorzugten Ausführungsform ist die Platte eine Rahmenplatte.

Zur Lösung der Aufgabe kann jede der oben genannten Platten einer Filterpresse als erfindungsgemäße Platte ausgeführt sein. Insbesondere ist die Ausführung der erfindungsgemäßen Platte als Filterrahmen zu bevorzugen, da dieser mit dem geringsten Aufwand und den geringsten Kosten herzustellen und folglich im Falle eines Versagens einfacher und kostengünstiger auszutauschen ist.

Vorteilhafterweise ist der Rahmen mit wenigstens einer Durchgangsbohrung für die Zu- und Abführung von Reinigungsmittel und wenigstens einer Durchgangsbohrung zur Entlüftung versehen. Die Durchgangsbohrungen erstrecken sich vorzugsweise senkrecht zur Plattenebene durch den Rahmen.

Vorzugsweise liegen die Durchgangsbohrungen in der umlaufenden Nut.

Die Anordnung einer Durchgangsbohrung im Rahmen und insbesondere in der umlaufenden Nut erlaubt die Zufuhr und die Abführung von Reinigungs-, Spül- und oder Sterilisiermittel insbesondere am reinigungskritischen Ort der Dichtung und im Anpressbereich zueinander benachbarter Platten.

Die Erfindung betrifft außerdem eine Filterpresse mit einer Mehrzahl parallel zueinander angeordneter Platten, wobei wenigstens jede zweite Platte eine Platte der oben beschriebenen Art ist.

Die Verwendung der Filterpresse ist insbesondere für die Filtration von Blutplasma oder für die Herstellung pharmazeutischer Produkte vorgesehen, weil die Filterpresse durch den Einsatz der erfindungsgemäßen Platten die höchsten Qualitätsstandards bezüglich Reinigung und Sterilisation erfüllt.

Weiterhin betrifft die Erfindung ein Verfahren zur Sterilisation einer vorstehend beschriebenen erfindungsgemäßen Filterpresse gemäß dem Anspruch 12.

Das Verfahren sieht folgende Verfahrensschritte vor:
Verfahren zur Reinigung der Platten einer Filterpresse gemäß Anspruch 12 umfassend folgende Verfahrensschritte:
(a) Erstes Zusammenpressen der Platten, wobei nur der zweite Vorsprung der Dichtung komprimiert wird,
(b) Mindestens ein Spülvorgang mit einem Reinigungs-, Spül- und/oder Sterilisationsmedium und
(c) Zweites Zusammenpressen der Platten, wobei sowohl der zweite als auch der erste Vorsprung der Dichtung vollständig komprimiert werden.

Mit diesem Reinigungsverfahren werden alle produktberührenden Bereiche gereinigt und/oder sterilisiert. Vorzugsweise wird die Filterpresse nach Durchführung des Reinigungsverfahrens bis zur Filtration nicht mehr geöffnet. Nach einer Sterilisation verbleibt die Sterilisationsflüssigkeit vorzugsweise in der Filterpresse, bis der Filtrationsbetrieb aufgenommen wird.

Vorzugsweise umfasst das Verfahren zur Reinigung der Platten einer Filterpresse folgende Verfahrensschritte:
(1) Auseinanderschieben der Plattenanordnung,
(2) Entfernen des Filterkuchens,
(3) Entnahme der Platten und separate Reinigung
(4) Einsetzen der gereinigten Platten
   (a) Erstes Zusammenpressen der Platten, wobei nur der zweite Vorsprung der Dichtung komprimiert wird,
   (b) Mindestens ein Spülvorgang mit einem Reinigungs-, Spül- und/oder Sterilisationsmedium und
   (c) Zweites Zusammenpressen der Platten, wobei sowohl der zweite als auch der erste Vorsprung der Dichtung vollständig komprimiert werden.

Das erfindungsgemäße Verfahren zur Reinigung einer Filterpresse sieht vor, dass nach einem abgeschlossenen Filterprozess die Platten der Filterpresse soweit auseinandergeschoben werden, dass der Filterkuchen abgelöst werden kann. Anschließend werden die Platten einzeln entnommen und separat gereinigt. Nach Wiedereinsetzen aller Platten in die Filterpresse werden diese wieder soweit zusammengepresst - erstes Zusammenpressen - bis die Platten an der Anschlagsfläche des ersten Vorsprungs der umlaufenden Dichtung der jeweils benachbarten Platte und ein Spalt entsteht, der gerade der Höhe des ersten Vorsprungs entspricht.

Anschließend wird die Filterpresse vergleichbar dem Filterbetrieb entsprechend im Reinigungsbetrieb unter der Verwendung von Reinigungs-, Spül- und/oder Sterilisationsmedien gefahren, wobei das Reinigungs-, Spül- und/oder Sterilisationsmedium mittels wenigstens einer Durchgangsbohrung im Rand der Platten dem in den Platten befindlichen Kammerraum zu- und abgeführt wird. Im Anschluss an den Spülbetrieb werden die Platten soweit zusammengepresst - zweites Zusammenpressen -, dass sowohl der erste als auch der zweite Vorsprung der Dichtung vollständig komprimiert werden und die Filterpresse bereit ist für einen neuen Filtrationsprozess.

Beispielhafte Ausführungsformen der Erfindung werden nachfolgend anhand der Figuren näher erläutert.

Es zeigen:
- Figur 1: eine perspektivische Darstellung einer Filterpresse,
- Figur 2: eine Draufsicht auf eine Rahmenplatte,
- Figur 3: eine Draufsicht auf eine aus dem Stand der Technik bekannte Membranplatte,
- Figur 4: eine Draufsicht auf eine aus dem Stand der Technik bekannte Filterplatte,
- Figur 5: ein Ausschnitt einer Plattenanordnung im Schnitt in geöffnetem Zustand der Filterpresse,
- Figur 6: ein Ausschnitt einer Plattenanordnung im Schnitt im Reinigungszustand der Filterpresse,
- Figur 7: ein Ausschnitt einer Plattenanordnung im Schnitt im Filterzustand der Filterpresse,
- Figur 8: ein Teilschnitt durch eine erfindungsgemäße Filterplatte, und
- Figur 9: ein Teilschnitt durch eine erfindungsgemäße Membranplatte.

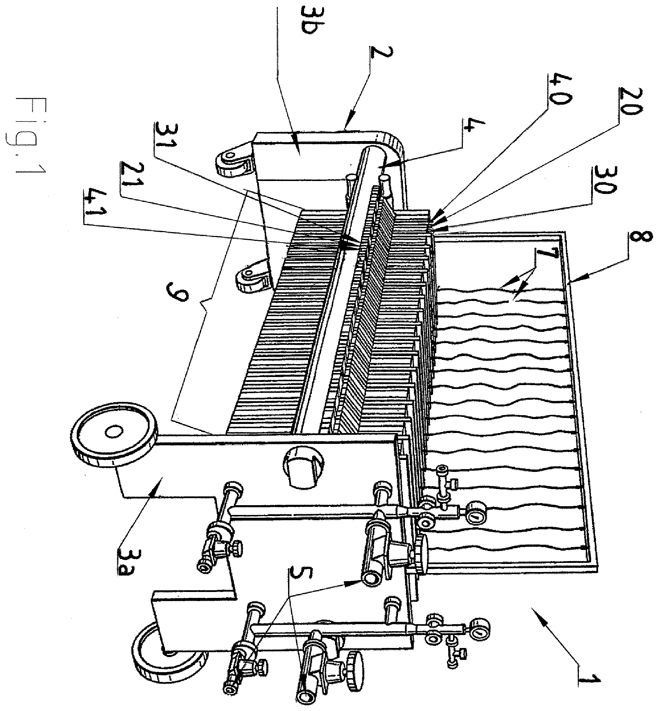

In der Figur 1 ist eine Filterpresse 1 perspektivisch dargestellt. Die Filterpresse 1 besitzt ein fahrbares Gestell 2, das an den beiden Enden jeweils eine Gestellplatte 3a, 3b aufweist, die über zwei Auflagebalken 4 miteinander verbunden sind. Auf diesen Auflagebalken 4 werden zur Bildung einer Plattenanordnung 9 die Rahmenplatten 20, Membranplatten 30 und die Filterplatten 40 in einer vorgesehenen Reihenfolge mit ihren Haltenasen 21, 31, 41 eingehängt und über eine Pressvorrichtung gegeneinander dichtend verpresst. Die Membranplatten 30 besitzen Druckluftanschlüsse 7', an denen Schläuche 7 befestigt sind, die über eine Stange 8 zu einer gemeinsamen Druckluftversorgung (nicht dargestellt) geführt werden.

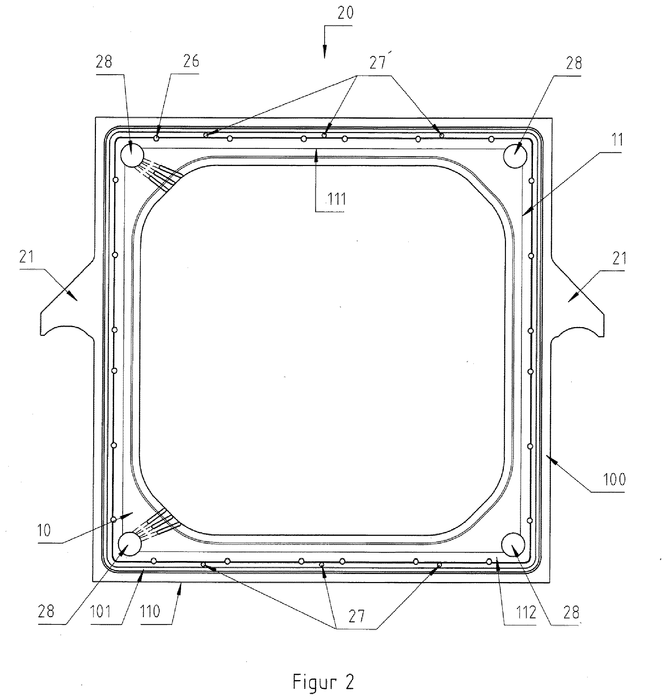

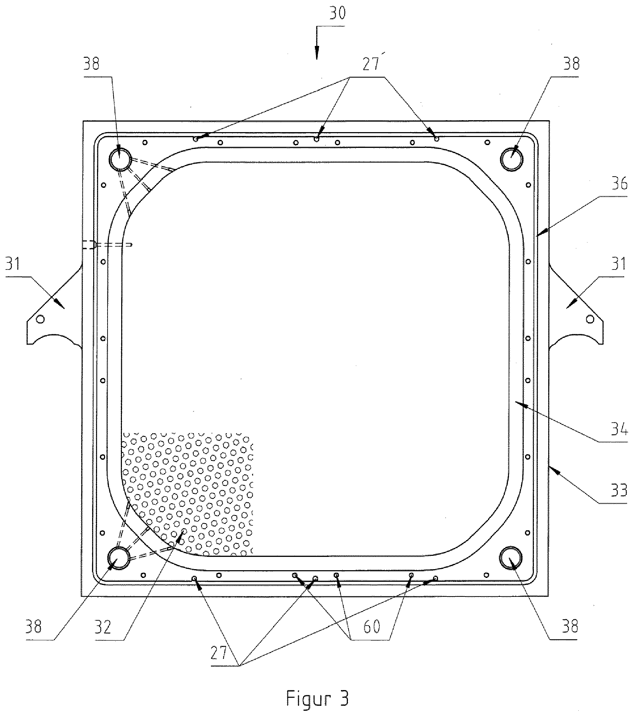

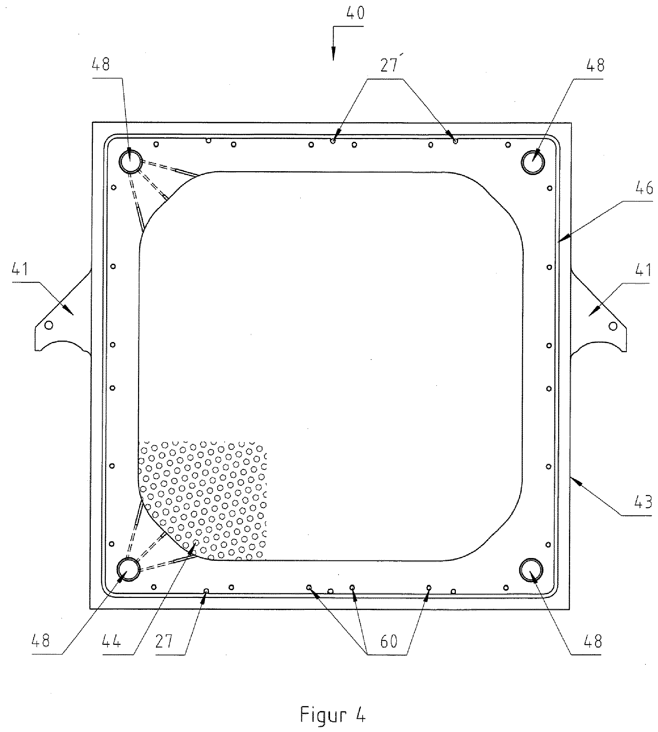

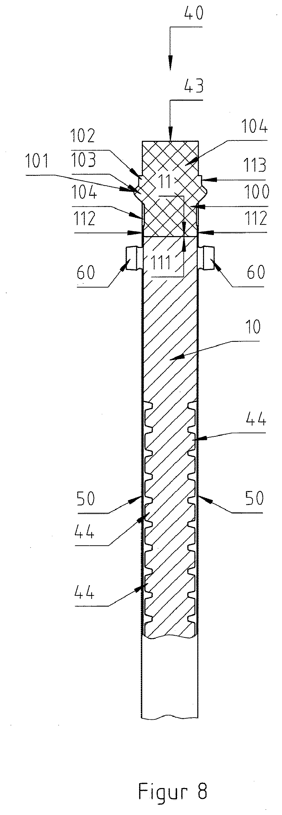

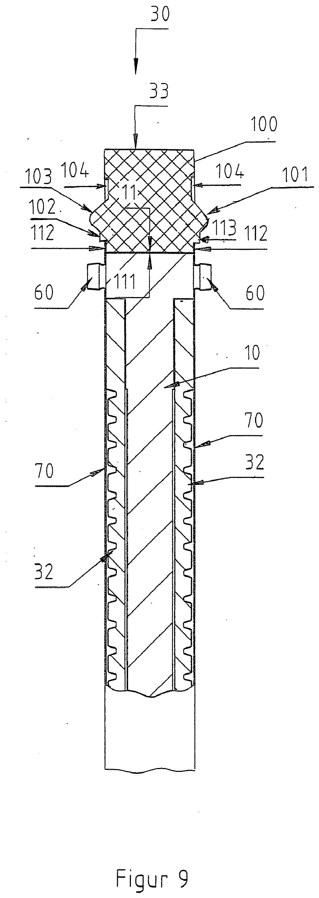

Im vorderen Teil des Gestells sind mehrere Anschlüsse 5 für Unfiltratzulauf und Filtratablauf vorgesehen, die mit den im Innern der Plattenanordnung 9 liegenden Filtrat- und Unfiltratkanälen verbunden sind. Zur Bildung dieser Filtrat- und Unfiltratkanäle sind in den Platten 20, 30 und 40 in den Eckbereichen entsprechend dimensionierte Augen 28, 38, 48 vorgesehen, die für oben genannte Plattenarten in den Figuren 2, 3 und 4 eingezeichnet sind. Die in Figuren 3 und 4 dargestellten Membranplatten- und Filter sind Platten nach dem Stand der Technik, die zusammen mit erfindungsgemäßen Rahmenplatten 20 verbaut werden. Die Figuren 8 und 9 zeigen Membran- und Filterplatten 30, 40 gemäß der erfindungsgemäßen Bauweise, die mit herkömmlichen Rahmenplatten, oder Rahmenplatten ohne die erfindungsgemäße Dichtung, zu einer Plattenanordnung zusammengesetzt werden können.

Die in Figur 2 dargestellte Rahmenplatte 20 setzt sich zusammen aus einem Grundkörper 10 und aus einem Material mit der Härte H₁ mit einer äußeren Umfangsfläche 11 und einem Rahmen 100 und aus einem Material H₂ mit einer äußeren Umfangsfläche 110, einer inneren Umfangsfläche 111 und zwei Seitenflächen 112. Der Rahmen 100 umschließt die äußere Umfangsfläche 11 des Grundkörpers 10, wobei die äußere Umfangsfläche 11 des Grundkörpers 10 mit der inneren Umfangsfläche 111 des Rahmens 100 verschweißt ist. An jeder Seitenfläche 112 des Rahmens 100 ist eine umlaufende, geschlossene Dichtung 101 angeordnet. Da es sich in Figur 2 um eine Draufsicht handelt, ist nur eine Seitenfläche 112 zu sehen.

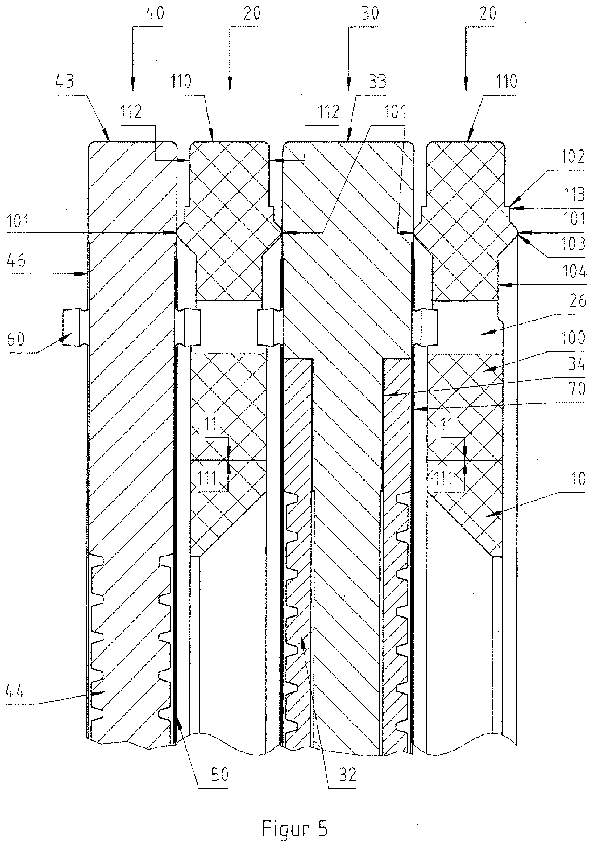

Wie in der Figur 5 zu sehen ist, ist die Dichtung 101 stufenförmig ausgebildet und weist im dargestellten unbelasteten Zustand einen ersten Vorsprung 102 und einen zweiten Vorsprung 103 auf, wobei beide Vorsprünge 102, 103 aneinandergrenzen und der zweite Vorsprung 103 den ersten Vorsprung 102 überragt. Der erste Vorsprung 102 liegt im dargestellten Ausführungsbeispiel näher an der äußeren Umfangsfläche 110 des Rahmens 100 als der zweite Vorsprung 103.

Innenseitig der Dichtung 101 verläuft eine Nut 104, welche unmittelbar an den zweiten Vorsprung 103 angrenzt und in das Rahmenmaterial gefräst sein kann. In der Nut 104 befinden sich Durchgangsbohrungen 27, welche zur Zu- und Abführung von Reinigungsmittel dienen (s. Figur 2, in Figur 5 nicht zu sehen). Die Durchgangsbohrungen 27 befinden sich in Bezug auf den Einbauzustand des Rahmens 20 im unteren Teil der umlaufenden Nut 104. Im oberen Teil der Nut 104 befinden sich Durchgangsbohrungen 27', welche zur Entlüftung des Kammerbereichs während des Reinigungsvorgangs dienen und gewährleisten, dass das Reinigungsmittel alle produktberührenden Bereiche erreicht.

Im dargestellten Beispiel weist die Rahmenplatte 20 zudem Bohrungen 26 im inneren Randbereich der umlaufenden Nut 104 auf, welche dazu dienen, Haltezapfen 60 benachbarter Membran- und Filterplatten 30, 40 aufzunehmen, welche zur Befestigung von Filtertüchern 50, 70 dienen (s. Figur 5).

Der Vollständigkeit halber zeigen die Figuren 3 und 4 eine Membranplatte 30 und eine Filterplatte 40, die bereits aus dem Stand der Technik bekannt sind und die in Verbindung mit der Rahmenplatte aus Figur 2 in einer erfindungsgemäßen Filterpresse 1 verwendet werden können.

In der Figur 3 ist eine Draufsicht auf eine solche Membranplatte 30 dargestellt, wobei das durch Haltezapfen 60 gehaltene Filtertuch 70 nicht dargestellt ist. Die Haltezapfen 60 sind beabstandet zur äußeren Umfangsfläche 33 der Membranplatte 30 angeordnet und liegen im Wesentlichen im Bereich der Verbindungslinie der Augen 38, die sich in den Eckbereichen der Membranplatte 30 befinden. Ferner ist eine Membran 32 zu sehen, die in einer Ausnehmung 34 liegt. Die Membranplatte 30 weist zudem eine Ringnut 36 auf, in der das Filtertuch 70 endet (s. auch Figur 5).

In der Figur 4 ist eine Filterplatte 40 dargestellt, die in ähnlicher Weise ausgebildet ist wie die Membranplatte 30 und ebenfalls Halteelemente 60 aufweist, die beabstandet zur äußeren Umfangsfläche 43 der Filterplatte 40 angeordnet sind (s. auch Figur 5). Auch hier ist eine Ringnut 46 vorgesehen, in der das Filtertuch 50 endet. Die Ausgestaltung der Ringnut 46 entspricht der der Ringnut 36. Ferner sind Augen 48 in den Eckbereichen der Filterplatte 40 vorgesehen. Der innere Bereich der Filterplatte 40 ist mit Noppen 44 ausgestattet, auf denen das nicht dargestellte Filtertuch 50 aufliegt.

Die Figuren 5 bis 7 zeigen jeweils einen Ausschnitt der Plattenordnung 9 in verschiedenen Betriebszuständen der Filterpresse 1 und sollen die Funktionsweise der Erfindung verdeutlichen.

Figur 5 zeigt die Plattenanordnung 9 mit Platten 20, 30, 40 im geöffneten Zustand der Filterpresse 1, wobei kein Anpressdruck auf die Platten 20, 30, 40 wirkt.

Die Plattenanordnung 9 weist Rahmenplatten 20, Membranplatten 30 und Filterplatten 40 auf, wobei sich Membran- und Filterplatten 30, 40 abwechseln und zwischen ihnen jeweils eine Rahmenplatte 20 mit oben beschriebenem Rahmen 100 und Dichtung 101 befindet.

Die Membranplatte 30 weist an ihren Seitenflächen jeweils eine Membran 32 auf, welche von einem Filtertuch 70 bedeckt ist. Das Filtertuch 70 ist mittels Haltezapfen 60 im Randbereich der Membranplatte 30 befestigt.

Die Seitenflächen der Filterplatte 40 sind ebenfalls jeweils mit einem Filtertuch bestückt, welche, wie auch im Fall der Membranplatte 30 mittels Haltezapfen 60 im Randbereich der Filterplatte 40 befestigt sind.

Die in den Figuren 5 bis 7 im Schnitt dargestellte Rahmenplatte 20 entspricht der Rahmenplatte 20 aus der Figur 2 und weist daher den Grundkörper 10 und den flexiblen Rahmen 100 auf. An den Seitenflächen 112 des Rahmens 100 sind die Dichtungen 101 angeordnet. Der erste Vorsprung 102 der Dichtung 101 weist eine zur Seitenfläche 112 parallele Anschlagfläche 113 auf. Der zweite Vorsprung 103 ist im dargestellten Beispiel höckerförmig ausgebildet.

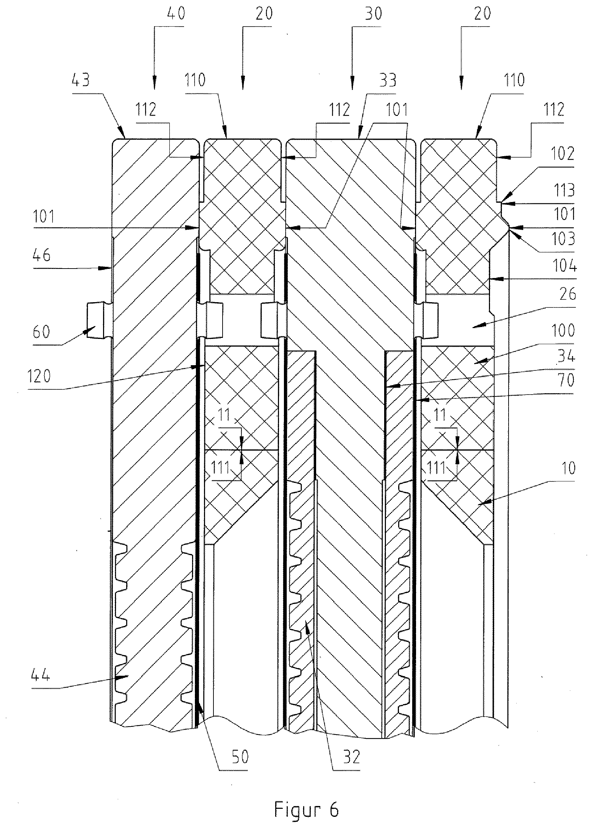

Die Figur 6 zeigt die Plattenanordnung 9 in der Zwischenposition, in der die Reinigung und Sterilisation der Platten 20, 30, 40 durchgeführt wird.

Für die Reinigung aller produktberührenden Bereiche der Platten 20, 30, 40 wird die Plattenanordnung 9 mit einem Anpressdruck der typischerweise zwischen 20 und 70 bar beträgt, zusammengepresst, bis die Membranplatte 30 und die Rahmenplatte 40 an den Anschlagsflächen 113 des ersten Vorsprungs 102 der Dichtungen 101 anliegen. Zwischen den Platten 20, 30, 40 werden Spalte 120 mit einer Breite von wenigen mm ausgebildet, in die das Sterilisationsmedium eindringen kann. Die Spaltbreite entspricht der Höhe des ersten Vorsprungs 102, der für die benachbarten Platten einen Anschlag bildet und aufgrund seines hohen Verformungswiderstands für eine gleichmäßige Anpressdruckfortpflanzung über die gesamte Plattenanordnung 9 hinweg sorgt.

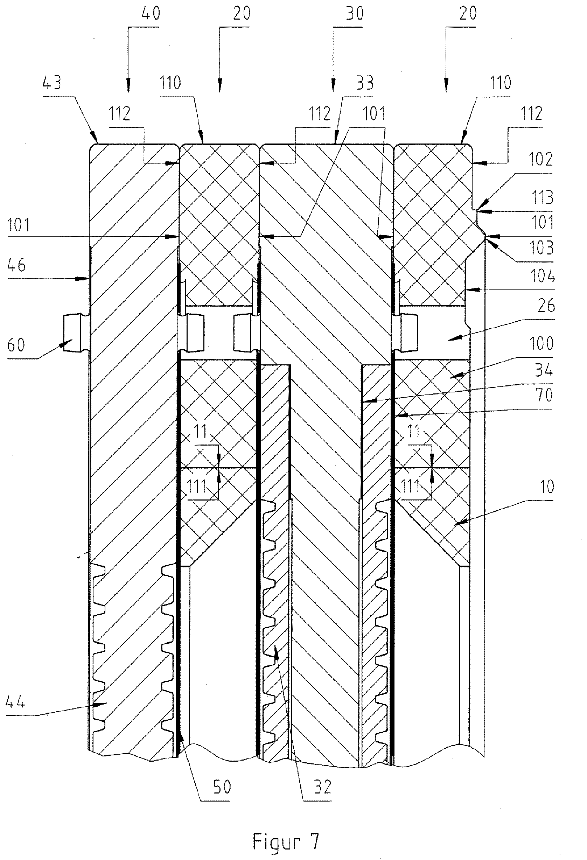

Figur 7 zeigt den Ausschnitt der Plattenanordnung 9 aus den Figuren 5 und 6 im Filterbetrieb. Die Platten werden mit einem hohen Anpressdruck von etwa 240 bar vollständig aneinander gepresst. Die flexible Dichtung 101 ist vollständig zusammengepresst, wobei das verdrängte Dichtungsmaterial von der Nut 104 aufgenommen wird.

In Figur 8 ist eine Filterplatte 40 mit den erfindungsgemäßen Merkmalen dargestellt. Die Filterplatte 40 weist einen Grundkörper 10 auf, der mit seiner äußeren Umfangsfläche 11 mit der inneren Umfangsfläche 111 des Rahmens 100 verschweißt ist. Der Grundkörper 10 besteht aus einem Material der Härte H₁ während das Material des Rahmens 100 eine Härte H₂ aufweist. Auch bei dieser Ausführungsform gilt H₂ < H₁. Die Haltezapfen 60 sind bei dieser Ausführungsform Bestandteil des Grundkörpers 10. Der Aufbau der gegenüber der Seitenfläche 112 vorstehenden Dichtung 101 entspricht der Ausgestaltung der Dichtung 101 gemäß der Rahmenplatte 20 gemäß Figur 5. Die Dichtung 101 weist einen ersten Vorsprung 102 und einen zweiten Vorsprung 103 auf, wobei der zweite Vorsprung 103 den ersten Vorsprung 102 überragt. Der erste Vorsprung 102 weist die parallele Anschlagsfläche 113 auf. Benachbart zum zweiten Vorsprung ist die Nut 104 angeordnet.

In der Figur 9 ist eine Membranplatte 30 mit den erfindungsgemäßen Merkmalen dargestellt. Die Membranplatte 30 weist einen Grundkörper 10 und einen Rahmen 100 auf. Die Haltezapfen 60 sind ebenfalls Bestandteil des Grundkörpers 10. Der Grundkörper 10 ist mit seiner äußeren Umfangsfläche 11 an der inneren Umfangsfläche 111 des Rahmens 100 verschweißt. Für die Materialien von Grundkörper 10 und Rahmen 100 werden dieselben Materialien wie bei den Grundkörpern 10 und Rahmen 100 der Filterplatte 40 und der Rahmenplatte 20 verwendet. Auch bei dieser Ausführungsform ist die Härte H₂ des Rahmens 100 kleiner als die Härte H₁ des Grundkörpers. An beiden Seitenflächen 112 ist jeweils eine erfindungsgemäße Dichtung 101 angeordnet, wobei die Reihenfolge von erstem Vorsprung 102 und zweitem Vorsprung 103 vertauscht ist. Dies bedeutet, dass der erste Vorsprung 102 innenliegend angeordnet ist und der zweite Vorsprung 103, der den Vorsprung 102 überragt, der Umfangsfläche 33 zugewandt ist. Dementsprechend ist auch die umlaufende Nut 104 zwischen der Umfangsfläche 33 und dem zweiten Vorsprung 103 angeordnet.

### Bezugszeichenliste

- 1: Filterpresse
- 2: fahrbares Gestell
- 3a, b: Gestellplatte
- 4: Auflagebalken
- 5: Anschlüsse
- 7: Schlauch
- 7': Druckluftanschluss
- 8: Stange
- 9: Plattenanordnung
- 10: Grundkörper
- 11: äußere Umfangsfläche

- 20: Rahmenplatte
- 21: Haltenase
- 26: Bohrung
- 27, 27': Durchgangsbohrung CIP
- 28: Auge

- 30: Membranplatte
- 31: Haltenase
- 32: Membran
- 33: äußere Umfangsfläche
- 34: Ausnehmung
- 36: Ringnut
- 38: Auge

- 40: Filterplatte
- 41: Haltenase
- 43: äußere Umfangsfläche
- 44: Noppen
- 46: Ringnut
- 48: Auge

- 50: Filtertuch

- 60: Haltezapfen

- 70: Filtertuch

- 100: Rahmen
- 101: Dichtung
- 102: erster Vorsprung
- 103: zweiter Vorsprung
- 104: Nut

- 110: äußere Umfangsfläche
- 111: innere Umfangsfläche
- 112: Seitenfläche
- 113: Anschlagfläche
- 120: Spalt

- H₁: Härte des Grundkörpers 10
- H₂: Härte des Rahmens 100

## Patentansprüche

1. Platte (20, 30, 40) für eine Filterpresse (1) **gekennzeichnet durch**
- einen Grundkörper (10), der aus einem ersten Material mit einer Härte H₁ besteht und eine äußere Umfangsfläche (11) aufweist, und
- einen an der äußeren Umfangsfläche (11) angeordneten Rahmen (100), der aus einem zweiten Material mit einer Härte H₂ besteht, wobei H₂ < H₁ ist, und
- wobei der Rahmen (100) an mindestens einer Seitenfläche (112) mindestens eine gegenüber der Seitenfläche (112) vorstehende, umlaufende Dichtung (101) aufweist.

2. Platte (20, 30, 40) nach Anspruch 1, **dadurch gekennzeichnet, dass** die Dichtung (101) und der Rahmen (100) aus einem Stück gefertigt sind.

3. Platte (20, 30, 40) nach Anspruch 1 oder 2, **dadurch gekennzeichnet, dass** der Rahmen(100) mit seiner inneren Umfangsfläche (111) mit der äußeren Umfangsfläche (11) des Grundkörpers (10) verbunden ist.

4. Platte (20, 30, 40) nach einem der vorstehenden Ansprüche, **dadurch gekennzeichnet, dass** die innere Umfangsfläche (111) des Rahmens (100) mit der äußeren Umfangsfläche (11) des Grundkörpers (10) verschweißt ist.

5. Platte nach einem der vorstehenden Ansprüche, **dadurch gekennzeichnet, dass** die umlaufende Dichtung (101) im unbelasteten Zustand einen ersten Vorsprung (102) aufweist, der in einen zweiten Vorsprung (103) übergeht.

6. Platte (20, 30, 40) nach Anspruch 5, **dadurch gekennzeichnet, dass** der erste Vorsprung (102) eine zur Seitenfläche (112) des Rahmens (100) parallel ausgerichtete Anschlagsfläche (113) aufweist.

7. Platte (20, 30, 40) nach einem der vorstehenden Ansprüche 5 oder 6, **dadurch gekennzeichnet, dass** der erste Vorsprung (102) näher an der äußeren Umfangsfläche (110) des Rahmens (100) angeordnet ist als der zweite Vorsprung (103).

8. Platte (20, 30, 40) nach einem der vorstehenden Ansprüche, **dadurch gekennzeichnet, dass** die Seitenfläche (112), die die Dichtung (101) aufweist, eine umlaufende Nut (104) aufweist, wobei die Nut (104) an den zweiten Vorsprung (103) angrenzt.

9. Platte (20, 30, 40) nach einem der vorstehenden Ansprüche, **dadurch gekennzeichnet, dass** die Platte (20, 30, 40) eine Filterplatte (40) ist oder dass die Platte (20, 30, 40) eine Membranplatte (30) ist oder dass die Platte (20, 30, 40) eine Rahmenplatte (20) ist.

10. Platte (20, 30, 40) nach einem der vorstehenden Ansprüche, **dadurch gekennzeichnet, dass** der Rahmen (100) wenigstens eine Durchgangsbohrung (27) für die Zu- und Abführung von Reinigungs- und/oder Sterilisationsmittel und wenigstens eine Durchgangsbohrung (27') zur Entlüftung aufweist.

11. Platte (20, 30, 40) nach Ansprüche 8 und 10, **dadurch gekennzeichnet, dass** die Durchgangsbohrungen (27, 27') in der umlaufenden Nut (104) angeordnet sind.

12. Filterpresse (1) mit einer Plattenanordnung (9), die eine Mehrzahl parallel zueinander angeordneter Platten (10, 20, 30) aufweist, **dadurch gekennzeichnet, dass** wenigstens jede zweite Platte eine Platte (20, 30, 40) gemäß einem der Ansprüche 1 bis 11 ist.

13. Verwendung einer Filterpresse nach Anspruch 12 für die Filtration von Blutplasma oder für die Herstellung pharmazeutischer Produkte.

14. Verfahren zur Reinigung der Platten (20, 30, 40) einer Filterpresse (1) gemäß Anspruch 12 umfassend folgende Verfahrensschritte:
(a) Erstes Zusammenpressen der Platten (20, 30, 40), wobei nur der zweite Vorsprung (103) der Dichtung (100) komprimiert wird,
(b) Mindestens ein Spülvorgang mit einem Reinigungs-, Spül- und/oder Sterilisationsmedium und
(c) Zweites Zusammenpressen der Platten (20, 30, 40), wobei sowohl der zweite als auch der erste Vorsprung (102, 103) der Dichtung (100) vollständig komprimiert werden.

15. Verfahren gemäß Anspruch 14 zur Reinigung der Platten einer Filterpresse (1) gemäß Anspruch 12, wobei den Verfahrensschritten (a), (b) und (c) folgende Schritte vorgeschaltet sind:
(1) Auseinanderschieben der Plattenanordnung (9),
(2) Entfernen des Filterkuchens,
(3) Entnahme der Platten (20, 30, 40) und separate Reinigung
(4) Einsetzen der gereinigten Platten (20, 30, 40)

## Claims

1. Plate (20, 30, 40) for a filter press (1), **characterised by**
- a base member (10) which comprises a first material having a hardness H₁ and which has an outer peripheral face (11), and
- a frame (100) which is arranged on the outer peripheral face (11) and which comprises a second material having a hardness H₂, wherein H₂ < H₁, and
- wherein the frame (100) has on at least one side face (112) at least one peripheral seal (101) which protrudes with respect to the side face (112).

2. Plate (20, 30, 40) according to claim 1, **characterised in that** the seal (101) and the frame (100) are produced in one piece.

3. Plate (20, 30, 40) according to claim 1 or claim 2, **characterised in that** the frame (100) is connected with the inner peripheral face (111) thereof to the outer peripheral face (11) of the base member (10).

4. Plate (20, 30, 40) according to any one of the preceding claims, **characterised in that** the inner peripheral face (111) of the frame (100) is welded to the outer peripheral face (11) of the base member (10).

5. Plate according to any one of the preceding claims, **characterised in that** the peripheral seal (101) in the unloaded state has a first projection (102) which merges into a second projection (103).

6. Plate (20, 30, 40) according to claim 5,
**characterised in that** the first projection (102) has a stop face (113) which is orientated parallel with the side face (112) of the frame (100).

7. Plate (20, 30, 40) according to either of the preceding claims 5 or 6,
**characterised in that** the first projection (102) is arranged closer to the outer peripheral face (110) of the frame (100) than the second projection (103).

8. Plate (20, 30, 40) according to any one of the preceding claims, **characterised in that** the side face (112) which has the seal (101) has a peripheral groove (104), wherein the groove (104) adjoins the second projection (103).

9. Plate (20, 30, 40) according to any one of the preceding claims, **characterised in that** the plate (20, 30, 40) is a filter plate (40) or **in that** the plate (20, 30, 40) is a membrane plate (30) or **in that** the plate (20, 30, 40) is a frame plate (20).

10. Plate (20, 30, 40) according to any one of the preceding claims, **characterised in that** the frame (100) has at least one through-hole (27) for the supply and discharge of cleaning and/or sterilising media and at least one through-hole (27') for ventilation.

11. Plate (20, 30, 40) according to claims 8 and 10, **characterised in that** the through-holes (27, 27') are arranged in the peripheral groove (104).

12. Filter press (1) having a plate arrangement (9) which has a plurality of plates (10, 20, 30) which are arranged parallel with each other, **characterised in that** at least every second plate is a plate (20, 30, 40) according to any one of claims 1 to 11.

13. Use of a filter press according to claim 12 for the filtration of blood plasma or for the production of pharmaceutical products.

14. Method for cleaning the plates (20, 30, 40) of a filter press (1) according to claim 12, comprising the following method steps:
(a) first pressing-together of the plates (20, 30, 40), wherein only the second projection (103) of the seal (100) is compressed,
(b) at least one flushing operation with a cleaning, flushing and/or sterilising medium, and
(c) second pressing-together of the plates (20, 30, 40), wherein both the second and the first projection (102, 103) of the seal (100) are completely compressed.

15. Method according to claim 14 for cleaning the plates of a filter press (1) according to claim 12, wherein the following steps take place prior to the method steps (a), (b) and (c):
(1) pushing apart the plate arrangement (9),
(2) removing the filter cake,
(3) removing the plates (20, 30, 40) and separate cleaning,
(4) inserting the cleaned plates (20, 30, 40).

## Revendications

1. Plateau (20, 30, 40) pour un filtre-presse (1), **caractérisé par**
- un corps de base (10), qui est constitué d'un premier matériau d'une dureté H₁ et qui présente une surface périphérique extérieure (11), et
- un cadre (100), qui est disposé sur la surface périphérique extérieure (11) et qui est constitué d'un deuxième matériau d'une dureté H₂, avec H₂ < H₁, et
- dans lequel le cadre (100) présente sur au moins une surface latérale (112) au moins un joint périphérique (101) en saillie par rapport à la surface latérale (112).

2. Plateau (20, 30, 40) selon la revendication 1, **caractérisé en ce que** le joint (101) et le cadre (100) sont réalisés d'une seule pièce.

3. Plateau (20, 30, 40) selon la revendication 1 ou 2, **caractérisé en ce que** le cadre (100) est relié par sa surface périphérique intérieure (111) à la surface périphérique extérieure (11) du corps de base (10).

4. Plateau (20, 30, 40) selon l'une des revendications précédentes, **caractérisé en ce que** la surface périphérique intérieure (111) du cadre (100) est soudée avec la surface périphérique extérieure (11) du corps de base (10).

5. Plateau selon l'une des revendications précédentes, **caractérisé en ce que** le joint périphérique (101) à l'état non chargé présente une première saillie (102) qui se prolonge par une deuxième saillie (103).

6. Plateau (20, 30, 40) selon la revendication 5, **caractérisé en ce que** la première saillie (102) présente une surface de butée (113) orientée parallèlement à la surface latérale (112) du cadre (100).

7. Plateau (20, 30, 40) selon l'une des revendications 5 ou 6 précédentes, **caractérisé en ce que** la première saillie (102) est disposée plus près de la surface périphérique extérieure (110) du cadre (100) que la deuxième saillie (103).

8. Plateau (20, 30, 40) selon l'une des revendications précédentes, **caractérisé en ce que** la surface latérale (112) qui présente le joint (101) présente une rainure périphérique (104), ladite rainure (104) étant adjacente à la deuxième saillie (103).

9. Plateau (20, 30, 40) selon l'une des revendications précédentes, **caractérisé en ce que** le plateau (20, 30, 40) est un plateau filtrant (40) ou que le plateau (20, 30, 40) est un plateau à membrane (30) ou que le plateau (20, 30, 40) est un plateau de cadre (20).

10. Plateau (20, 30, 40) selon l'une des revendications précédentes, **caractérisé en ce que** le cadre (100) présente au moins un trou traversant (27) pour l'amenée et l'évacuation d'un produit de nettoyage et/ou de stérilisation et au moins un trou traversant (27') pour la ventilation.

11. Plateau (20, 30, 40) selon les revendications 8 et 10, **caractérisé en ce que** les trous traversants (27, 27') sont disposés dans la rainure périphérique (104).

12. Filtre-presse (1) doté d'un agencement de plateaux (9) qui présente plusieurs plateaux (10, 20, 30) disposés parallèlement les uns aux autres, **caractérisé en ce qu'**au moins un plateau sur deux est un plateau (20, 30, 40) selon l'une des revendications 1 à 11.

13. Utilisation d'un filtre-presse selon la revendication 12 pour la filtration de plasma sanguin ou pour la fabrication de produits pharmaceutiques.

14. Procédé de nettoyage des plateaux (20, 30, 40) d'un filtre-presse (1) selon la revendication 12, comprenant les étapes de procédé suivantes :
(a) première compression des plateaux (20, 30, 40), seule la deuxième saillie (103) du joint (100) étant comprimée,
(b) au moins un processus de rinçage avec un agent de nettoyage, de rinçage et/ou de stérilisation, et
(c) deuxième compression des plateaux (20, 30, 40), la deuxième et la première saillie (102, 103) du joint (100) étant toutes deux complètement comprimées.

15. Procédé selon la revendication 14 de nettoyage des plateaux d'un filtre-presse (1) selon la revendication 12, dans lequel les étapes de procédé (a), (b) et (c) sont précédées des étapes suivantes :
(1) écartement de l'agencement de plateaux (9),
(2) enlèvement du gâteau de filtration,
(3) enlèvement des plateaux (20, 30, 40) et nettoyage séparé
(4) insertion des plateaux nettoyés (20, 30, 40).
